# EUROPÄISCHE PATENTSCHRIFT

(11) **EP 1 364 674 B1**
(45) Veröffentlichungstag und Bekanntmachung des Hinweises auf die Patenterteilung: **14.02.2007**
(21) Anmeldenummer: 02011525.9
(22) Anmeldetag: 23.05.2002
(51) Int. Cl.: A61M 16/14

(54) **Verfahren und Antriebseinheit zum Antrieb eines medizinischen Gerätes mit komprimiertem Gas**
Method and drive unit to drive a medical device by means of compressed gas
Méthode et moteur pour faire marcher un appareil médical par gaz comprimé

(43) Veröffentlichungstag der Anmeldung: 26.11.2003
(73) Patentinhaber: Tecnostore AG, 6003 Luzern (CH)
(72) Erfinder: Geisser, Albert, 6003 Luzern (CH); Kezmann, Bruno Rudolf, 6048 Horw (CH)
(74) Vertreter: Blum, Rudolf Emil

(56) Entgegenhaltungen:
- EP-A- 0 998 954
- WO-A-94/23788
- DE-A- 3 011 621
- DE-C- 900 783
- US-A- 3 360 168
- US-A- 4 973 247
- PATENT ABSTRACTS OF JAPAN vol. 2002, no. 06, 4. Juni 2002 (2002-06-04) & JP 2002 059044 A (UISUTA:KK), 26. Februar 2002 (2002-02-26)
- PATENT ABSTRACTS OF JAPAN vol. 1999, no. 03, 31. März 1999 (1999-03-31) & JP 10 323392 A (MEDEITETSUKU JAPAN:KK), 8. Dezember 1998 (1998-12-08)

## Beschreibung

Die Erfindung betrifft ein Verfahren zum Antrieb eines medizinischen, insbesondere orthopädischen Spülgerätes. Ferner betrifft die Erfindung ein medizinisches Spülgerät gemäss Anspruch 11.

Medizinische Geräte werden z.B. elektrisch oder pneumatisch angetrieben, was entsprechende Leitungen und Schläuche bedingt. Diese können die Handhabung der Geräte erschweren und müssen regelmässig gereinigt und gewartet werden, was aufwendig ist. JP-A-2002 059044 zeigt ein Spülgerät gemäss Oberbegriff der vorliegenden Erfindung. EP-A-998 954 zeigt die Förderung eines Duftstoffes mit einem Gasstrom und US-A-3 350 168 zeigt die Förderung einer Flüssigkeit mit einem Gas. WO 94/23788 zeigt die Förderung von Feststoffteilchen mittels eines Gasstromes, wobei im Förderteil durch Turbulenz das Verklumpen von Feststoffteilchen vermieden werden soll. JP-A-10323392 zeigt einen Zerstäuber zur Vernebelung einer Medizin, wobei mit einem Gasstrom aus einem Gasvorrat die Förderung und Vernebelung der Medizin bewirkt wird.

Der Erfindung liegt die Aufgabe zugrunde, ein verbessertes Verfahren zum Antrieb eines medizinischen Spülgerätes zu schaffen.

Dies wird mit den Merkmalen des Anspruchs 1 erreicht.

Dadurch, dass im Spülgerät ein die Flüssigkeit fördernder Gasstrom aus einem Gasvorrat erzeugt wird, kann auf eine Zuleitung für Antriebsenergie verzichtet werden; der Mischer bewirkt eine besonders gute Mischung zwischen dem Gas, vorzugsweise CO₂, und der Flüssigkeit.

Bevorzugterweise sind der Gasvorratsbehälter und auch das Mittel zur Erzeugung des Gasstromes austauschbar am Gerät angeordnet. Dies ermöglicht die Gestaltung dieser Elemente als einmal verwendbare Einheit, die nach Verwendung entsorgt werden kann; das Gerät ist mit einer neuen Einheit erneut verwendbar. Vorzugsweise ist die Einheit dabei als steril verpackte Einheit ausgestaltet.

Das Spülgerät kann auch die Spülflüssigkeit in einem Behälter im oder am Gerät enthalten, insbesondere in einem austauschbaren Behälter, so dass auch für die Spülflüssigkeit kein Schlauch zum Gerät führt.

Weiter liegt der Erfindung die Aufgabe zugrunde, ein medizinisches Spülgerät zu schaffen, das die genannten Nachteile nicht aufweist.

Diese Aufgabe wird durch ein Spülgerät gemäss Anspruch 11 gelöst.

Es ist bevorzugt, wenn das Mittel zur Erzeugung des Gasstromes vom Spülgerät abkoppelbar ist. Damit kann das Gerät nach Erschöpfung des Gasvorrates durch einfaches Ankoppeln eines neuen Mittels weiter betrieben werden.

Im folgenden werden Ausführungsbeispiele der Erfindung anhand der Zeichnungen näher erläutert. Dabei zeigt
Figur 1 schematisch ein Spülgerät;
Figur 2 schematisch eine Antriebseinheit für das Gerät im Ruhezustand;
Figur 3 die Antriebseinheit von Figur 2 im an das Gerät angekoppelten Zustand;
Figur 4 das Entsperren der Antriebseinheit; und
Figur 5 die Erzeugung des Gasstromes in der Antriebseinheit.

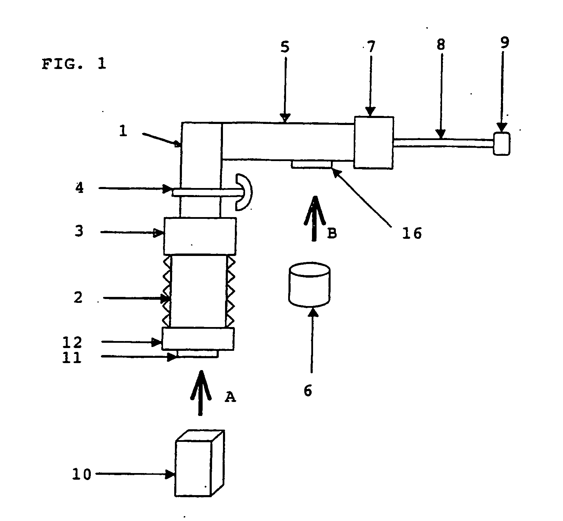

Figur 1 zeigt schematisch eine Ansicht eines Spülgerätes 1 gemäss der Erfindung.

Das Spülgerät ist dabei als Handgerät gezeigt, welches einen Handgriff 2 aufweist und ein von Hand betätigbares Ventil 4, welches die Abgabe von Spülflüssigkeit auslöst bzw. beendet. Die Spülflüssigkeit wird dabei in dem gezeigten Beispiel über ein Rohr 8 an eine Düse 9 abgegeben, wo die Spülflüssigkeit austritt. Diese Ausgestaltung des Spülflüssigkeitsaustritts ist natürlich nur als Beispiel zu verstehen und kann auf vielfältige bekannte Weise modifiziert werden. Das Gerät kann z.B. verwendet werden, um den Kanal im Knochen bei Hüftgelenksprothesen-Operationen zu spülen. Dies ist natürlich nur ein Beispiel, und das Spülgerät kann auch für andere Anwendungen verwendet werden, wo eine Abgabe von Spülflüssigkeit notwendig ist. Vorzugsweise befindet sich die Spülflüssigkeit in einem auswechselbaren Behälter 6, der über eine Kupplung 16 an das Gerät 1 ankoppelbar und wieder lösbar ist. Der Behälter kann dabei eine Kochsalzlösung enthalten und zum Beispiel als steril verpackter, einmal verwendbarer Behälter ausgestaltet sein, der nach der Entleerung entsorgt wird. Das Gerät 1 kann zur Entleerung des Behälters eine Saugpumpe 5 oder ein anderes Mittel aufweisen, welches die Flüssigkeit aus dem Behälter 6 hinaus und in das Rohr 8 bzw. aus der Düse 9 fördert. Die Förderung erfolgt dabei mittels eines Gasstromes, dessen Erzeugung nachfolgend erläutert wird. Der Gasstrom kann in der Saugpumpe 5 zum Ansaugen der Flüssigkeit aus dem Behälter 6 verwendet werden, oder es kann der Gasstrom die Förderung so bewirken, dass er Flüssigkeit aus dem Behälter 6 hinausdrückt. Solche Mittel zur Förderung bzw. zum Ansaugen oder Hinauspressen einer Flüssigkeit mittels einer Gasströmung sind dem Fachmann wohlbekannt und werden hier nicht im Detail erläutert. Es ist ferner ein Mischer 7 vorgesehen, welcher eine Mischung zwischen dem Gas des Gasstromes und der geförderten Flüssigkeit bewirkt. Eine Mischung wird sich zwar je nach Art der Förderung im Teil 5 bereits dort zwischen der Flüssigkeit und dem Gasstrom ergeben, der Mischer 7 ist jedoch noch zusätzlich vorgesehen, um eine besonders gute Mischung zwischen Gas, vorzugsweise CO₂, und der Flüssigkeit herbeizuführen. Zu diesem Zweck kann auch z.B. nur ein Teil des Gasstromes zunächst im Element 5 zur Förderung verwendet werden und ein anderer Teil des Gasstromes wird direkt dem Mischer zugeführt und dort erst mit der geförderten Flüssigkeit in Kontakt gebracht. Auch solche Mischelemente 7 sind dem Fachmann bekannt und werden hier nicht im Detail erläutert. Die mit Gas, insbesondere CO₂, gemischte Flüssigkeit kann, verglichen mit Flüssigkeit ohne Gasbeimischung, eine besonders gute Spülwirkung erzielen. CO₂ weist keine schädlichen Wirkungen beim Spülen eines Knochenkanals auf; im Gegensatz zu Luft besteht keine Emboliegefahr.

Gemäss der Erfindung wird nun der Gasstrom, der das Gerät antreibt bzw. bei dem Spülgerät die Förderung der Flüssigkeit bewirkt, im Gerät 1 selber erzeugt. Zu diesem Zweck ist im Gerät 1 ein Gasgenerator vorgesehen, der z.B. im Handgriff 2 angeordnet sein kann. Der Gasgenerator kann dabei auf die nachfolgend erläuterte Weise ausgestaltet sein. Der Gasgenerator kann dabei einen wiederbefüllbaren Gasvorratsbehälter aufweisen, welcher das Wiederbefüllen des Gerätes 1 mit Gas erlaubt. Der Gasvorratsbehälter kann auch austauschbar sein, so dass nach Erschöpfung des Gasvorrates ein neuer Gasvorratsbehälter eingesetzt wird. Je nach Art der Gaserzeugung bzw. des erzeugten Gasdruckes kann ein Druckreduzierventil 3 vorgesehen sein, welches einen möglichst konstanten Gasdruck an seinem Ausgang bereithält. Der in das Förderelement 5 bzw. das Mischelement 7 geführte Gasstrom weist dann einen im wesentlichen konstanten Druck auf, was bevorzugt ist. Im folgenden wird eine besonders bevorzugte Ausführungsart erläutert, bei welcher der das Gerät antreibende Gasstrom vermittels eines Gasstromerzeugungsmittels 10 erzeugt wird, welches an das Gerät ankoppelbar ist. Die für das Mittel 10 gemachten Ausführungen gelten indes auch, wenn die Gaserzeugung auf die vorgenannte Weise im Gerät selber, z.B. im Handgriff 2, erfolgt. Das Mittel 10 ist dabei an das Gerät 1 ankoppelbar, wozu eine Kupplung 11 am Gerät vorgesehen ist. Das Mittel 10 weist dann ein entsprechendes Kupplungsteil auf, mit welchem die Kopplung an das Gerät 1 bewirkt werden kann. Nach Erschöpfung des Gasvorrates kann das Mittel 10 vom Gerät 1 entfernt werden und für den weiteren oder nächsten Gebrauch durch ein neues Mitel 10 ersetzt werden. Eine Entkupplungssperre 12 kann vorgesehen sein, welche auf noch zu erläuternde Weise das Abkoppeln des Gasstromerzeugungsmittels während der Erzeugung des Gasstromes sperrt und auch die Erzeugung des Gasstromes erst erlaubt, wenn das Mittel 10 am Gerät 1 angekoppelt ist. Das Mittel 10 ist dabei in Figur 1 nur schematisch als Quader dargestellt, es kann aber natürlich eine beliebige Form aufweisen. Die Ankopplung erfolgt in der Schemazeichnung von Figur 1, in dem das Mittel 10 in Richtung des Pfeiles A an das Gerät 1 gedrückt und dort mit der Kupplung 11 befestigt wird. Dasselbe ist für den Behälter 6 für die zu fördernde Flüssigkeit mit dem Pfeil B angedeutet, der das Befestigen des Behälters 6 an der Kupplung 16 darstellen soll.

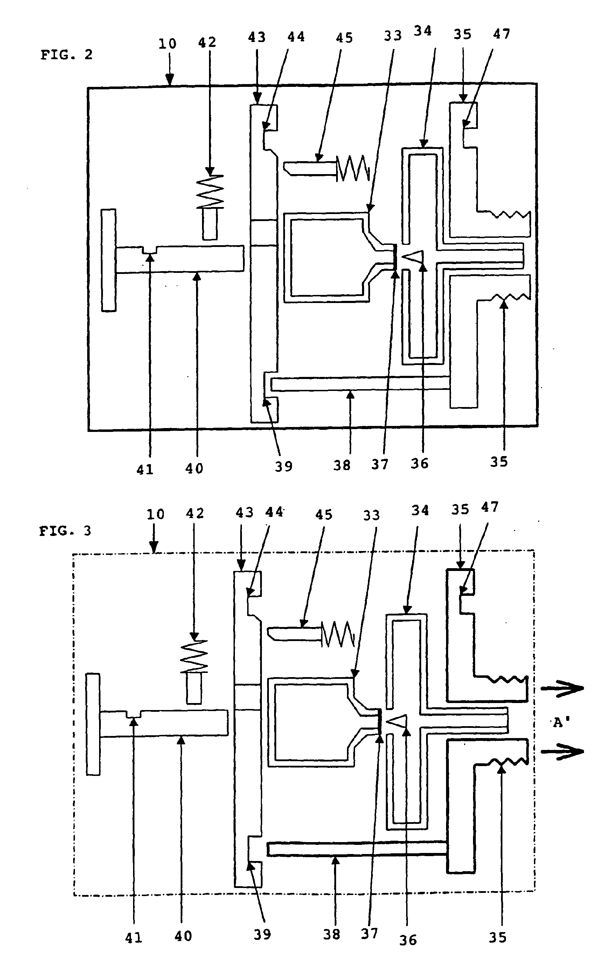

Die Figur 2 zeigt nun eine stark schematisierte genauere Ansicht des Gasstromerzeugungsmittels 10. Dieses weist einen Gasvorratsbehälter 33 auf, welcher ein an sich bekannter patronenförmiger Behälter sein kann, der ein Gas, vorzugsweise CO₂, in flüssiger Form enthält. Solche Gaspatronen sind handelsüblich. Die Gaspatrone ist mit einer Membrane 37 oder einem Ventil verschlossen. Bevorzugt wird eine Gaspatrone mit CO₂ verwendet, da dieses bei einer medizinischen Verwendung in der Regel unschädlich ist. Grundsätzlich könnte die Gaspatrone indes auch Luft enthalten oder Stickstoff. Das Gas kann in der Patrone auch unter hohem Druck in nichtverflüssigter Form gespeichert sein.

In dem gezeigten Beispiel weist das Mittel 10 weiter eine Kupplung 35 auf, mittels welchem es an das Gerät 1 angekoppelt werden kann. Dies kann z.B. eine Kupplung mit einem Schraubgewinde sein, die ein Anschrauben des Mittels 10 am Gerät 1 bzw. dessen Kupplungsteil 11 erlaubt. Natürlich kann auch eine andere Art der Kupplung verwendet werden. Die Kupplung 35 kann über einen Freigabebolzen 38 mit einer Sicherungsplatte 43 verbunden sein, welche in der gezeigten Stellung verhindert, dass ein Betätigungselement 40 die Gaspatrone 33 in Richtung auf einen Einstichdorn 36 bewegen kann, um die Gasfreigabe zu bewirken. Dieser Sicherungsmechanismus wird nachfolgend noch genauer beschrieben. Natürlich ist dabei das schematisch gezeigte Beispiel nur als eine von verschiedenen möglichen Ausführungsvarianten zu verstehen.

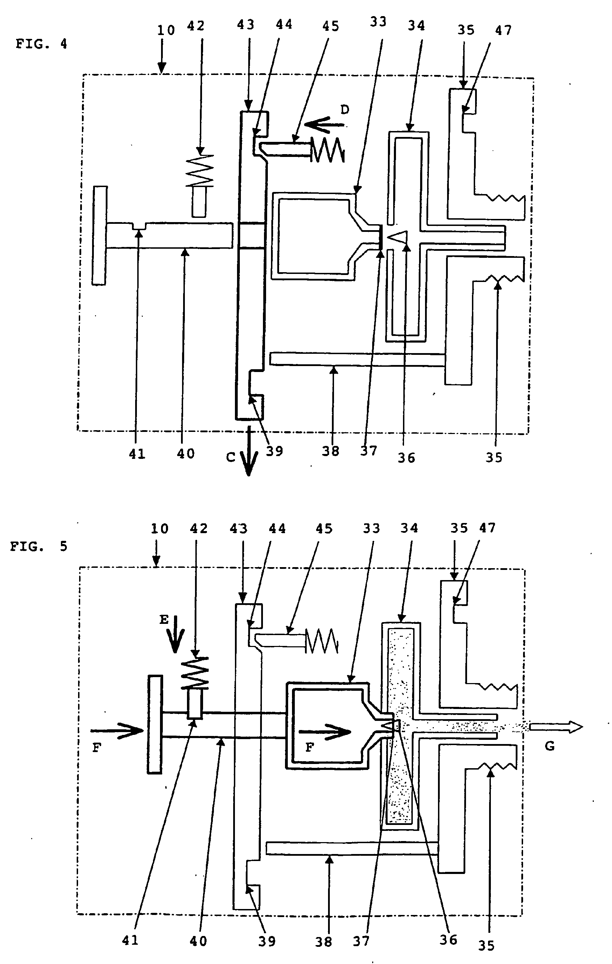

Figur 3 zeigt nun in der selben stark schematisierten Form das Mittel 10 in an das Gerät 1 angekoppelter Stellung. Dabei bezeichnen gleiche Bezugszeichen wie bis anhin verwendet die gleichen Elemente. Dies gilt auch für die weiteren Figuren. Durch die Ankopplung des Mittels 10 an das Gerät 1 ist die Kupplung 35 in Richtung des Pfeiles A' bewegt worden, z.B. indem das nur angedeutete Gehäuse des Mittels 10 am Gerät 1 zum Anliegen gekommen ist und das Hineinschrauben der Kupplung 35 in ein gegengleiches Gewinde des Gerätes 1 die Kupplung 35 zum Gerät hin bewegt hat. Dadurch ist der Freigabebolzen 38 aus der Ausnehmung 39 der Sicherungsplatte 43 herausbewegt worden. Die Sicherungsplatte 43 ist dadurch nun in der Figur 3 nach unten verschiebbar. Figur 4 zeigt, wie entsprechend die Sicherungsplatte 43 in Richtung des Pfeiles C bewegt worden ist, was z.B. von Hand durch Druck auf ein aus dem Gehäuse vorstehendes Teil der Sicherungsplatte 43 bewirkt werden kann. Die Sicherungsplatte 43 kann in der in Figur 4 gezeigten Freigabestellung durch einen Sicherungsbolzen 45 arretiert werden, der in eine weitere Ausnehmung 44 der Sicherungsplatte eingreift. Dabei bewegt sich der Sicherungsbolzen 45 in Richtung des Pfeiles D. Diese Sicherung kann so ausgeführt sein, dass sie eine Zurückbewegung der Sicherungsplatte 43 in die Stellung von Figur 34 erlaubt, falls das Mittel nun doch nicht in Betrieb genommen werden soll.

Soll dies nun jedoch geschehen, so wird, wiederum von Hand, auf das Betätigungselement 40 ein Druck in Richtung des Pfeiles F ausgeübt, was in Figur 5 gezeigt ist. Das Betätigungselement 40 kann in der Stellung von Figur 4 der Sicherungsplatte 43 durch eine Ausnehmung derselben hindurchtreten und die Gaspatrone 33 in Richtung auf den Einstichdorn 36 bewegen, was die Membrane 37 zerstört oder das Ventil öffnet, so dass der Inhalt der Gaspatrone 33 freigegeben wird. Das Betätigungselement 40 kann dabei durch eine sich in Pfeilrichtung E bewegende Arretierung 42 in der betätigten Stellung definitiv arretiert werden, so dass nach Betätigung des Elementes 40 eine definierte Lage der Gaspatrone 33 erzielt wird, welche nicht mehr verändert werden kann. Dies wird bevorzugt, um ein Austreten von Gas der einmal geöffneten Patrone in eine andere als der gewollten Richtung zu verhindern .

Figur 5 zeigt, wie die aufgestochene Gaspatrone 33 mit einem Verdampfer 34 in Verbindung ist. In diesem Verdampfer wird das vorzugsweise flüssige Gas der Patrone unter Wärmezufuhr verdampft, wobei der Verdampfer die Wärme vorzugsweise aus der Umgebung aufnimmt und somit nicht aktiv beheizt wird. Entsprechende Verdampfer sind grundsätzlich bekannt und bestehen im wesentlichen aus einem grossflächigen Wärmetauscher, welcher die Umgebungswärme an das flüssige Gas abgibt, so dass dieses verdampfen kann. Für den Fall, dass das Gas in gasförmiger Form in der Patrone gespeichert ist, ist natürlich kein Verdampfer vorgesehen, sondern allenfalls ein erstes Druckreduzierventil, welches den Gasstrom, der aus dem Mittel 10 austritt, in seinem Druck auf gewünschte Weise reduziert. Im gezeigten Beispiel der Figuren 4-6 tritt der Gasstrom in Richtung des Pfeiles G aus dem Verdampfer aus und gelangt durch entsprechende, nicht dargestellte dichtende Anschlussstücke der Kupplung 11 in das Gerät 1, wo er zum Antrieb des Gerätes weitergeleitet und benutzt wird. Bei dem in Figur 1 gezeigten Gerät kann dazu, wie bereits erwähnt, ein Druckreduzierventil 3 im Gerät vorgesehen sein. Ferner ein betätigbares Sperrventil 4, welches den Gasstrom wahlweise durchlassen oder unterbrechen kann, um das Gerät einzuschalten oder auszuschalten.

Das ankoppelbare Mittel 10, in welchem der Gasstrom erzeugt wird, ergibt ein sehr flexibles Antriebskonzept, welches natürlich bei beliebigen Geräten vorgesehen sein kann. Bei medizinischen Geräten ist es bevorzugt, wenn das Mittel 10 als steril verpacktes Mittel vorliegt, welches bei Bedarf an das Gerät 1 angekoppelt werden kann. Ist der Gasvorrat des Mittels 10 erschöpft, so wird dieses vom Gerät 1 gelöst und kann entsorgt oder recycliert werden oder es kann allenfalls eine Wiederbefüllung durch Einsetzen einer neuen Gaspatrone durchgeführt werden. Wird das Gerät 1 weiter benötigt, so kann ein neues Mittel 10 angekoppelt werden. Wird das Gerät 1 nicht mehr benötigt, so kann es gereinigt werden und bei einer späteren Verwendung kann wiederum ein Mittel 10 angekoppelt werden. Das Sicherheitsmerkmal, welches die Auslösung des Gasstromes erst bei angekoppeltem Gerät erlaubt, ist dabei bevorzugt, um eine erhöhte Sicherheit zu geben, indem eine unkontrollierte Gasabgabe verhindert wird. Die Kupplung 35 des Mittels 10 kann ferner so ausgestaltet sein, dass sie eine Abkopplung des Mittels 10 erst erlaubt, wenn diese drucklos geworden ist bzw. der Vorrat in der Patrone 33 erschöpft ist. Zu diesem Zwecke kann ein Eingriffsglied einer Entkupplungsperre 12 des Gerätes vorgesehen sein, welches z.B. in eine Ausnehmung 47 der Kupplung 35 des Mittels 10 eingreift solange die Sperre 12 einen Gasdruck feststellt, derart, dass bei Vorhandensein des Gasdruckes durch die Kupplung 35 eine Abkopplung des Mittels 10 nicht möglich ist. Diese bisher genannten Sicherungsmechanismen sind bevorzugt, aber sind natürlich nur fakultativ vorhanden.

## Patentansprüche

1. Verfahren zum Antrieb eines medizinischen, insbesondere orthopädischen, Spülgerätes (1) zur Abgabe einer Flüssigkeitsströmung, wobei im Spülgerät (1) aus einem Gasvorratsbehälter (33) ein Gasstrom erzeugt wird, der als Antriebsmittel wirksam ist, und mittels des Gasstromes und einem Förderteil (5) eine Spülflüssigkeit gefördert wird, **dadurch gekennzeichnet, dass** die geförderte Spülflüssigkeit in einem dem Förderteil (5) in Strömungsrichtung nachgeschalteten Mischteil (7) mit dem Gasstrom vermischt wird, wobei der Gasstrom aufgeteilt wird und ein erster Teil im Förderteil (5) zur Förderung der Flüssigkeit verwendet wird und ein weiter Teil des Gasstromes direkt dem Mischteil (7) zugeführt und erst dort mit der Flüssigkeit zur Mischung mit der geförderten Flüssigkeit in Kontakt gebracht wird.

2. Verfahren nach Anspruch 1, **dadurch gekennzeichnet, dass** mittels eines CO₂-Gasstromes Kochsalzlösung als Spülflüssigkeit gefördert und mit dem CO₂-Gasstrom vermischt wird.

3. Verfahren nach Anspruch 1 oder 2, **dadurch gekennzeichnet, dass** der Gasvorratsbehälter (33) zusammen mit dem Gasstromerzeugungsmittel (10) austauschbar am Spülgerät (1) angekoppelt ist.

4. Verfahren nach einem der Ansprüche 1 bis 3, **dadurch gekennzeichnet, dass** der Gasstrom durch das Verdampfen eines in verflüssigter Form gespeicherten Gases erzeugt wird.

5. Verfahren nach einem der Ansprüche 1 bis 4, **dadurch gekennzeichnet, dass** das Gas in einer Einwegpatrone oder einer wiederbefüllbaren Patrone gespeichert ist, welche zur Erzeugung des Gasstromes entleert wird.

6. Verfahren nach Anspruch 5, **dadurch gekennzeichnet, dass** die Patrone und vorzugsweise auch das Gasstromerzeugungsmittel eine steril verpackte, zur einmaligen Verwendung bestimmte Einheit bilden

7. Verfahren nach einem der Ansprüche 1 bis 6, **dadurch gekennzeichnet, dass** das Gasstromerzeugungsmittel einen Sicherheitsmechanismus aufweist, der eine unbeabsichtigte Auslösung des Gasstromes verhindert.

8. Verfahren nach Anspruch 7, **dadurch gekennzeichnet, dass** der Gasstrom nur auslösbar ist, wenn das Gasstromerzeugungsmittel am Spülgerät angekoppelt worden ist.

9. Verfahren nach Anspruch 7 oder 8, **dadurch gekennzeichnet, dass** das Gasstromerzeugungsmittel vom Spülgerät nur trennbar ist, wenn der Gasvorrat erschöpft ist.

10. Verfahren nach Anspruch 5, **dadurch gekennzeichnet, dass** ein Sicherheitsmechanismus vorgesehen ist, der die Trennung von Gaspatrone und Verdampfer nach deren Verbindung verhindert, solange der Gasvorrat nicht erschöpft ist.

11. Medizinisches Spülgerät (1) zur Abgabe eines Flüssigkeitsstromes, umfassend einen Gasvorrat (33) und ein Mittel (10) zur Erzeugung eines Gasstromes aus dem Vorrat sowie einen Vorratsbehälter (6) für Flüssigkeit, einen vom Gasstrom antreibbaren Förderteil (5) für die Flüssigkeit und einen Flüssigkeitsaustritt (9), **dadurch gekennzeichnet, dass** das Spülgerät (1) weiter einen dem Förderteil (5) nachgeschalteten Mischteil (7) für die Mischung der geförderten Flüssigkeit mit einem Teil des Gasstromes aufweist, und dass der Gasstrom aufgeteilt ist und ein erster Teil im Förderteil (5) zur Förderung der Flüssigkeit eingesetzt ist und ein zweiter Teil des Gasstromes direkt dem Mischteil (7) zuführbar und erst dort mit dem Flüssigkeitsstrom in Kontakt bringbar ist.

12. Medizinisches Spülgerät nach Anspruch 11, **dadurch gekennzeichnet, dass** das Mittel (10) zur Erzeugung des Gasstromes über ein Kopplungsmittel (11) lösbar am Gerät befestigt ist.

13. Medizinisches Spülgerät nach Anspruch 11, **dadurch gekennzeichnet, dass** als Gasvorratsbehälter eine Gaspatrone, insbesondere enthaltend CO₂, vorgesehen ist, und dass das Gasstromerzeugungsmittel einen mit der Gaspatrone unter Öffnung derselben durch ein Auslösemittel in Verbindung bringbaren Verdampfer oder ein Druckreduzierventil mit einem Austritt für den Gasstrom aufweist.

## Claims

1. A method for driving of a medical rinsing apparatus (1), in particular an orthopaedic rinsing apparatus, for delivering a liquid stream, wherein a gas stream from a gas reservoir (33) is generated within the rinsing apparatus, which works as driving means, and a rinsing liquid is fed by said gas stream and a feeding part (5) **characterized in that** the fed rinsing liquid is mixed with the gas stream in a mixing part (7) which is arranged downstream of the feeding part (5), wherein the gas stream is divided and a first part thereof is used in the feeding part (5) for feeding the liquid and a second part of the gas stream is led directly to the mixing part (7) and is brought only there in contact for mixing with the fed liquid.

2. A method according to claim 1, **characterized in that** saline solution as rinsing liquid is fed by a CO₂ gas stream and is mixed with the CO₂ gas stream.

3. A method according to claims 1 or 2 **characterized in that** the gas reservoir (33) together with the gas stream generating means (10) is exchangeably coupled to the rinsing apparatus (1).

4. A method according to one of claims 1 to 3, **characterized in that** the gas stream is generated by evaporating of a gas stored in liquid form.

5. A method according to one of claims 1 to 4, **characterized in that** the gas is stored in a single serving capsule or in a refillable capsule, which is emptied for generating the gas stream.

6. A method according to claim 5 **characterized in that** the capsule, and preferably the gas stream generating means as well, form an aseptic packed unit dedicated for single use.

7. A method according to one of claims 1 to 6 **characterized in that** the gas stream generating means comprises a security mechanism preventing an accidental release of the gas stream.

8. A method according to claim 7 **characterized in that** the gas stream is only releasable if the gas stream generating means is coupled to the rinsing apparatus.

9. A method according to claim 7 or 8 **characterized in that** the gas stream generating means is only detachable from the rinsing apparatus if the gas reservoir is depleted.

10. A method according to claim 5, **characterized in that** a security mechanism is provided that prevents disconnection of the connected gas capsule and the evaporator as long as the gas reservoir is not depleted.

11. A medical rinsing apparatus (1) for delivering a stream of a liquid, comprising a gas reservoir (33) and a means (10) for generating a gas stream from said reservoir and a reservoir (6) for a liquid, a feeding part (5) for the liquid drivable by the gas stream and a liquid exit (9) **characterized in that** the rinsing apparatus (1) further comprises a mixing part (7) downstream of the feeding part (5) for mixing the fed liquid with a part of the gas stream, and **in that** the gas stream is divided and a first part is used in the feeding part (5) for feeding the liquid and a second part of the gas stream is feedable directly to the mixing part (7) and is bringable into contact with the liquid stream only there.

12. A medical rinsing apparatus according to claim 11 **characterized in that** the means (10) for generating the gas stream is detachably fixed to the apparatus by a coupling means (11).

13. A medical rinsing apparatus according to claim 11 **characterized in that** a gas capsule is provided as gas reservoir, in particular a capsule containing CO₂, and that the gas stream generating means comprises an evaporator that is brought in connection with the capsule by a releasing means while opening the capsule, or a pressure reducing valve with an opening for the gas stream.

## Revendications

1. Procédé pour l'actionnement d'un dispositif de lavage (1) médical, en particulier orthopédique, destiné à distribuer un flux de liquide, un flux de gaz étant généré dans le dispositif de lavage (1) à partir d'une réserve de gaz (33) et agissant comme un moyen de propulsion, et un liquide de lavage étant transporté au moyen du flux de gaz et d'un élément de transport (5), **caractérisé en ce que** le liquide de lavage transporté est mélangé au flux de gaz dans un mélangeur (7), qui est monté en aval de l'élément de transport (5) par référence à la direction d'écoulement, le flux de gaz étant séparé et une première partie étant utilisée dans l'élément de transport (5) pour le transport du liquide et une deuxième partie du flux de gaz étant acheminée directement vers le mélangeur (7) et, seulement dans celui-ci, étant amenée en contact avec le liquide pour être mélangée au liquide transporté.

2. Procédé selon la revendication 1, **caractérisé en ce qu'**une solution de sel de cuisine, formant le liquide de lavage, est transportée au moyen d'un flux de gaz CO₂ et est mélangé avec le flux de gaz CO₂.

3. Procédé selon la revendication 1 ou 2, **caractérisé en ce que** la réserve de gaz (33), conjointement avec l'élément destiné à générer le flux de gaz (10), est couplée de manière amovible au dispositif de lavage (1).

4. Procédé selon l'une quelconque des revendications 1 à 3, **caractérisé en ce que** le flux de gaz est généré par la vaporisation d'un gaz stocké sous forme fluidifiée.

5. Procédé selon l'une quelconque des revendications 1 à 4, **caractérisé en ce que** le gaz est stocké dans une cartouche à usage unique ou dans une cartouche rechargeable, qui est vidée pour générer le flux de gaz.

6. Procédé selon la revendication 5, **caractérisé en ce que** la cartouche et, de préférence aussi, l'élément destiné à générer le flux de gaz forment une unité conditionnée dans un emballage stérile et destinée à un usage unique.

7. Procédé selon l'une quelconque des revendications 1 à 6, **caractérisé en ce que** l'élément destiné à générer le flux de gaz comporte un mécanisme de sécurité qui empêche un déclenchement accidentel du flux de gaz.

8. Procédé selon la revendication 7, **caractérisé en ce que** le flux de gaz ne peut être déclenché que si l'élément destiné à générer le flux de gaz a été couplé au dispositif de lavage.

9. Procédé selon la revendication 7 ou 8, **caractérisé en ce que** l'élément destiné à générer le flux de gaz ne peut être séparé du dispositif de lavage que lorsque la réserve de gaz est épuisée.

10. Procédé selon la revendication 5, **caractérisé en ce qu'**il est prévu un mécanisme de sécurité qui empêche que, après leur assemblage, la cartouche de gaz et le vaporisateur soient séparés l'un de l'autre, tant que la réserve de gaz n'est pas épuisée.

11. Dispositif de lavage (1) médical pour distribuer un flux de liquide, comportant une réserve de gaz (33) et un moyen (10) pour générer un flux de gaz à partir de la réserve, ainsi qu'un réservoir (6) pour liquide, un élément de transport (5) pour le liquide, apte à être propulsé par le flux de gaz, et une sortie de liquide (9), **caractérisé en ce que** le dispositif de lavage (1) comporte, en outre, un mélangeur (7) qui est monté en aval de l'élément de transport (5) et qui est destiné à mélanger le liquide transporté avec une partie du flux de gaz, et **en ce que** le flux de gaz est séparé, une première partie étant introduite dans l'élément de transport (5) pour transporter le liquide et une deuxième partie du flux de gaz pouvant être acheminée directement vers le mélangeur (7) et, seulement dans celui-ci, peut être amenée en contact avec le flux de liquide.

12. Dispositif de lavage médical selon la revendication 11, **caractérisé en ce que** l'élément (10) destiné à générer un flux de gaz est fixé de manière amovible sur le dispositif par l'intermédiaire d'un moyen de couplage (11).

13. Dispositif de lavage médical selon la revendication 11, **caractérisé en ce que** la réserve de gaz prévue est une cartouche de gaz, contenant en particulier du CO₂, et **en ce que** le moyen pour générer le flux de gaz comporte un vaporisateur, pouvant être relié à la cartouche de gaz moyennant l'ouverture de celle-ci par l'intermédiaire d'un moyen de déclenchement, ou une soupape de diminution de pression avec une sortie pour le flux de gaz.
